(19) Europäisches Patentamt
European Patent Office
Office européen des brevets

(11) **EP 2 661 211 B1**

(12) **EUROPEAN PATENT SPECIFICATION**

(45) Date of publication and mention
of the grant of the patent:
**30.03.2022 Bulletin 2022/13**

(21) Application number: **12705722.2**

(22) Date of filing: **05.01.2012**

(51) International Patent Classification (IPC):
**A61B 1/00** *(2006.01)*      **A61B 1/04** *(2006.01)*

(52) Cooperative Patent Classification (CPC):
**A61B 1/00009; A61B 1/00039; A61B 1/00167;
A61B 1/00172; A61B 1/00188; A61B 1/042;**
A61B 1/00096

(86) International application number:
**PCT/IL2012/050004**

(87) International publication number:
**WO 2012/093401 (12.07.2012 Gazette 2012/28)**

(54) **IMAGING SYSTEM AND METHOD USING MULTICORE FIBER**

ABBILDUNGSSYSTEM UND -VERFAHREN MIT EINER MEHRKERNFASER

SYSTÈME ET PROCÉDÉ D'IMAGERIE UTILISANT UNE FIBRE À PLUSIEURS COEURS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **05.01.2011   US 201161457116 P**

(43) Date of publication of application:
**13.11.2013 Bulletin 2013/46**

(73) Proprietor: **Bar-Ilan University
52900 Ramat-Gan (IL)**

(72) Inventors:
• **ZALEVSKY, Zeev
48560 Rosh HaAyin (IL)**

• **SHAHMOON, Asaf
49773 Petach Tikva (IL)**
• **SLOVIN, Hamutal
76885 Moshav Galia (IL)**

(74) Representative: **Pearl Cohen Zedek Latzer Baratz
UK LLP
The Gridiron Building
One Pancras Square
London N1C 4AG (GB)**

(56) References cited:
**US-A1- 2009 316 116     US-A1- 2010 046 897
US-A1- 2010 274 090     US-A1- 2010 282 954**

## Description

## FIELD OF THE INVENTION

The present invention relates generally to the domain of imaging systems and methods.

## REFERENCES

The following references belong to the technical background of the present invention:

[1] G. Unfried1, F. Wieser, A. Albrecht, A. Kaider and F. Nagele, "Flexible versus rigid endoscopes for outpatient hysteroscopy: a prospective randomized clinical trial" Human Reproduction 16, 168-171 (2001).

[2] R. P. J. Barretto, B. Messerschmidt and M. J. Schnitzer, "In vivo fluorescence imaging with highresolution microlenses," Nature methods 6, 511-514 (2009).

[3] B. A. Flusberg, E. D. Cocker, W. Piyawattanametha, J. C. Jung, E. L. M. Cheung and M. J. Schnitzer, "Fiber-optic fluorescence imaging," Nature methods 2, 941-950 (2005).

[4] M. E. Llewellyn, R. P. J. Barretto, S. L. Delp and M. J. Schnitzer, "Minimally invasive high-speed imaging of sarcomere contractile dynamics in mice and humans," Nature 454, 784-788 (2008).

[5] K. Deisseroth, G. Feng, A. K. Majewska, G. Miesenbock, A. Ting and M. J. Schnitzer, "Next-Generation Optical Technologies for Illuminating Genetically Targeted Brain Circuits," The Journal of Neuroscience, 26, 10380 -10386 (2006).

[6] B. A. Flusberg, J. C. Jung, E. D. Cocker, E. P. Anderson and M. J. Schnitzer, "In vivo brain imaging using a portable 3.9 gram two-photon fluorescence microendoscope," Opt. Lett. 30, 2272-2274 (2005).

[7] W. Piyawattanametha, E. D. Cocker, L. D. Burns, R. P. J. Barretto, J. C. Jung, H. Ra, O. Solgaard and M. J. Schnitzer, "In vivo brain imaging using a portable 2.9g two-photon microscope based on a microelectromechanical systems scanning mirror," Opt. Lett. 34, 2309- 2311 (2009).

[8] Z. Zalevsky and D. Mendlovic, Optical Super Resolution, Springer (2004).

[9] A. Borkowski, Z. Zalevsky and B. Javidi, "Geometrical Super Resolved Imaging Using Non periodic Spatial Masking," JOSA A 26, 589-601 (2009).

[10] J. Fortin, P. Chevrette, and R. Plante "Evaluation of the microscanning process," SPIE Vol. 2269, Infrared Technology XX, Bjorn F. Andresen, Editors, 271-279 (1994).

[11] V. Mico, Z. Zalevsky and J. Garcia, "Common-path Phase-shifting Digital Holographic Microscopy: a way to Quantitative Phase Imaging and Superresolution," Opt. Commun. 281, 4273-4281 (2008).

## BACKGROUND OF THE INVENTION

Endoscopes are the common medical instrumentation to perform medical inspection of internal organs. There are two main types of endoscopes: flexible and rigid. The flexible endoscopes are being constructed out of a bundle of single mode fibers while each fiber in the bundle transmits backwards spatial information corresponding to a single spatial point, i.e. a single pixel. The fibers bundle may go into the body while the imaging camera is located outside. Interface optics adapts the photonic information coming out of the bundle to the detection camera. The reason for using single mode fiber for each fiber in the bundle rather than multi mode fibers (capable of transmitting spatial information that is corresponding to plurality of pixels) is related to the fact that when inserting the endoscope and while navigating it inside the body it may be bent. When multi mode fibers are bent the spatial modes are coupled to each other and the image is strongly distorted. The typical diameter of a single mode fiber in the bundle is about $30\mu m$ (this is the diameter of its cladding, the core has diameter of about $8-9\mu m$). The typical number of fibers in the bundle is about 10,000-30,000. Typical overall diameter (of the entire bundle) is about 3mm-5mm.

Another type of endoscopes is called rigid endoscope. In this case the camera going inside the body of the patient rather than staying outside while it is located on the edge of a rigid stick. Although image quality of rigid endoscopes is usually better and they allow not only backwards transmission of images but also other medical treatment procedures, their main disadvantage is related to the fact that they are indeed rigid and thus less flexible and less adapted for in-body navigation procedures.

There are alternative solutions to endoscopy which for instance involve pills swallowed by the patient and capable of capturing images of internal organs while propagated through the stomach and the intestine.

Multi core fibers were also proven to be suitable to perform high quality imaging tasks. In Refs. [2-4] one may see an overview of the state of the art of *in vivo* fluorescence imaging with high resolution micro lenses. In Refs. [5-7] one may see the demonstration of this micro endoscope for in vivo brain fluorescence imaging application. The use of multicore fibers might be preferred in invasive applications as it minimizes damage due to the small diameter of such an instrument.

For example, an endoscope utilizing a multicore fiber is described in US Patent Application US-A1-2010/0046897 which discloses an endoscope system including an image fiber with an image fiber main body made of a plurality of cores for forming pixels and a cladding common thereto; and an optical system connected to an eyepiece side of the image fiber for causing laser light to enter the image fiber and for taking in an image from the image fiber, in which the image fiber has the cores arranged substantially uniformly over a cross-section of the image fiber main body, the cross-section being perpendicular to a longitudinal direction of the image fiber main body.

United States Patent Application Publication 2010/274090 discloses an endoscope which includes an elongated tube. An objective lens system is disposed in the elongated tube, for passing image light from an object. A fiber optic image guide includes plural optical fibers bundled together, has a distal tip, is inserted through the elongated tube, for transmitting the image light focused on the distal tip by the objective lens system in a proximal direction. A displacing device displaces the distal tip laterally and periodically upon receiving entry of the image light being focused by use of a piezoelectric actuator positioned outside the distal tip. A support casing supports the distal tip inserted therein, keeps the distal tip shiftable on the displacing device, and transmits force of the piezoelectric actuator disposed outside to the fiber optic image guide.

United States Patent Application Publication 2010/282954 discloses an optical probe with an optical guide, e.g. an optical fibre, and a lens system rigidly coupled to an end portion of the optical guide. The probe has a housing with a cavity for the optical guide, the housing having at its distal end a transparent window, the window having an insignificant optical power as compared to the optical power of the said lens system. Actuation means displaces the lens system so as to enable optical scanning of a region of interest (ROI). The invention is particularly suited for miniature applications e.g. for in-vivo medical application. By attaching the lens system to the optical guide via the mount, the field of view (FOV) of the optical probe may be determined directly by the transverse stroke of the optical fibre. Hence only a relatively small stroke is required. The field of view is thus effectively no longer limited by the transverse stroke. The optical probe is especially advantageous for non-linear optical imaging where the optical guide may be an optical fibre with a relatively low exit numerical aperture. Another system for imaging an object with a shifting multicore optical fiber is disclosed in US 2008/081950 A1.

## GENERAL DESCRIPTION

The use of multicore fibers is advantageous in various applications, including medical applications, because of their small size and a possibility of making the instrument desirably flexible, if needed. However, multicore fiber based imaging faces issues related to limited resolution and/or field of view when used for imaging in near field or in far field conditions.

The present invention provides with a novel imaging system and method enabling to overcome these limitations. The imaging system of the invention includes a multicore fiber forming or being part of an optical imaging unit for imaging an object on a detection array. The multicore fiber by its input edge faces an object plane and by its output edge faces the detector plane. The multicore fiber thus collects light from the object at the input edge and transfers collected light to the output edge. Further provided in the imaging system is a displacing unit which operates to provide at least a lateral shift of the input edge of the multicore fiber relatively to the object, i.e. in a plane substantially perpendicular to the optical axis of the optical imaging unit. By this, a set of shifted images of the object can be sequentially obtained at the detector array. The displacing unit is controllably operated by an operating unit which sets a shifting amplitude to be either a first amplitude inferior or equal to the diameter of a core in the multicore fiber or a second amplitude superior or equal to the diameter of the multicore fiber.

As will be described further below, when imaging using a near field mode, the spatial resolution might be sufficiently high, however, the field of view is generally limited. By laterally shifting the multicore fiber with the second shifting amplitude, the field of view in the combined image formed by multiple successively acquired images, can be improved. On the other hand, when imaging an object using a far field mode, the field of view might be sufficient but the spatial resolution is typically limited. This can be solved, or at least partially solved, by using the lateral shift of the multicore fiber with the first shifting amplitude.

The invention provides for utilizing both the lateral shift of the multicore fiber, as described above, and also a longitudinal shift of the system operation between the far and near field modes. The latter can be implemented by either shifting the input edge of the multicore fiber itself, or alternatively (or additionally) using an imaging optics movable along the optical axis of the optical imaging unit. Practically, it would be easier to locate such a lens between the multicore fiber and the detector array, but generally a movable lens may be between the object and the multicore fiber.

The present invention is defined in the appended claims.

## BRIEF DESCRIPTION OF THE DRAWINGS

In order to understand the invention and to see how it may be carried out in practice, embodiments will now be described, by way of non-limiting example only, with reference to the accompanying drawings, in which:

> **Fig. 1** is a general diagram illustrating a system for imaging an object according to an embodiment of the present invention.
> **Fig. 2** is a general diagram illustrating a method for imaging an object according to an embodiment of the present invention.
> **Fig. 3** is a picture of a probe of a system according to an embodiment of the present invention.
> **Fig. 4** is an image of an output edge of a probe of a system imaging an object according to an embodiment of the present invention.
> **Figs. 5A-5B** illustrate experimental results of images acquired by an imaging system according to an embodiment of the present invention.

**Fig. 6** illustrates experimental results of images of Fe beads imaged through an agar solution with an imaging system according to an embodiment of the present invention.

**Figs. 7A-7B** illustrate experimental results of imaging a rat heart muscle growth on a slide with a microscope (Fig. 7A) and with an imaging system according to an embodiment of the present invention (Fig. 7B).

**Figs. 8A-8D** illustrate experimental results of imaging of blood veins in a chicken wing with a microscope (Fig. 8A) and with an imaging system according to an embodiment of the present invention (Figs. 8B-8D).

**Fig. 9** illustrates experimental results of imaging along a blood vein of a chicken wing with an imaging system according to an embodiment of the present invention.

**Fig. 10** illustrates an experimental setup with an imaging system for imaging a rat brain according to an embodiment of the present invention.

**Fig. 11** illustrates experimental results of imaging blood vessels inside a brain of a rat with an imaging system according to an embodiment of the invention.

**DETAILED DESCRIPTION OF EMBODIMENTS**

The present invention proposes an imaging system that includes a multicore fiber (also referred to as a probe) containing tens of thousands of cores properly separated to avoid optical leakage between them even when bended (e.g. when navigated through a body). The structure of the probe allows performing resolution enhancement, i.e. super resolution based on shifts of an input tip of the probe. The structure of the probe also enables to perform field of view enhancement based on shifts of the input tip. Further, the optical cores of the multicore fiber act to transmit backwards a wave front and to generate an image, however one or more of the cores may also be used to illuminate the object itself or even to heat the object if illuminated with high photonic power density. Furthermore, illuminating the object with coherent light such as laser may allow extraction of 3D information by interference configuration near the detection plane in which not only the amplitude but also the phase of the reflected wave front can be estimated For example, an active reference beam at the detector array plane may be interfered with a wave front reflected by the object and transferred through the multicore fiber, thereby enabling to obtain phase information on the wave front reflected by the object. The phase information may enable to obtain 3D information on the object i.e. to build a profile of the object. In another embodiment, the interference configuration may be replaced by an holographic setup.

The probe allows realization of an optical operation equivalent to optical zooming, i.e. reducing the field of view and increasing the sampling resolution. This operation may be obtained by axially shifting an optical assembly (i.e. moving from the far field regime where we have large field of view and lower resolution into the near field approximation where we have good resolution and small field of view).

The cross section of the probe may be rectangular thereby allowing to coat two of its opposite faces with metals to realize electrical stimulation capability at its edge including heating/cooling or thermal sensing based upon the Pelletier effect when two different types of metals are used for the coating.

The proposed probe can be used for large variety of new biomedical applications in which its thin diameter allows noninvasive medical operability. The applications may involve navigation through blood artery, going through the tears holes into internal chambers in the nose and the head, performing navigation through lambs especially those of small children (having smaller channels) and performing prostate as well as womb related medical treatments.

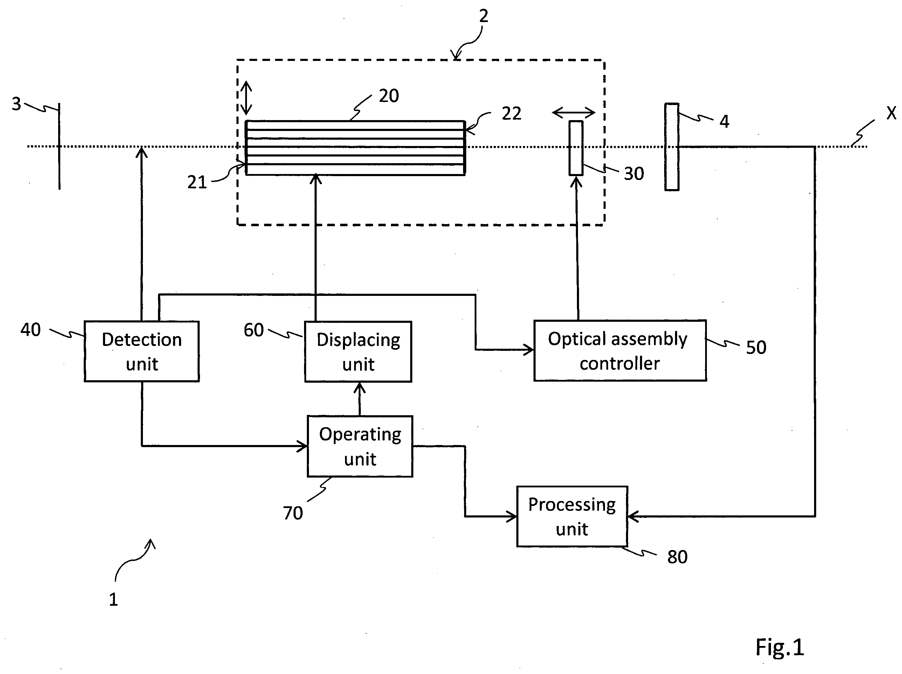

**Fig. 1** illustrates generally an imaging system **1** according to an embodiment of the present invention. The imaging system **1** comprises an optical imaging unit **2** configured to form an image of an object **3** on a detector array **4** of the imaging system 1. The object **3** may be positioned far from the imaging unit **2** i.e. so that an optical wave front emitted by the object **3** and arriving to the imaging unit **2** may be considered as a plane wave front (far field approximation). The object **3** may alternatively be positioned close to the imaging unit **2** so that the previous far field approximation is not valid. Hereinafter, these two configurations are respectively referred to as far field and near field arrangements (or configuration, modes, etc.).

The optical imaging unit **2** defines an optical axis **X** and comprises a multicore fiber **20** (also referred to as a probe) and an optical assembly **30**. The multicore fiber **20** may transfer light arriving from the object **3** to an input edge **21** of the multicore fiber **20** toward an output edge **22** of the multicore fiber **20**. The optical assembly **30** may be configured to collect light at the output edge **22** of the multicore fiber **20** and to focus the light collected on the detector array **4**. The optical assembly **30** may be configured to form images of the object **3** either positioned in the far field or in the near field of the imaging unit **2** i.e. relatively far or relatively close of the input edge **21**. In an embodiment, the optical assembly **30** may be adaptable with regard to the position of the object **3** to be imaged on the detector array **4**. In an embodiment, the optical assembly **30** may be an imaging lens positioned between the output edge **22** and the detector array **4**. In said embodiment, the imaging lens may be longitudinally displaceable along the optical axis **X** so as to accommodate light from an object **3** positioned either in the far field or in the near field. In another embodiment, the optical assembly **30** may comprise an input lens positioned upstream of the input edge **21**. The diameter of a core and the diameter of the multicore fiber **20** may be respectively referred to as *d* and *D.* The values of *d* and *D* are defined by fabrication and application related limitations. For example, D may be smaller than 300μm in order to remain non invasive in certain medical applications. The value of d may be determined according to a desired spatial resolution. *If D* is equal to 300μm and one wishes to have 100x100 pixels resolution it means that *d* may be about 3μm. Generally, *d* may be larger than an optical wavelength of the light collected in order to allow coupling of light to the fiber with sufficient energetic efficiency.

The imaging system **1** may further comprise a detection unit **40,** an optical assembly controller **50,** a displacing unit **60,** an operating unit **70** and a processing unit **80.** The detection unit **40** may be configured to monitor the position of the object **3** with regard to the input edge **21.** For example, the detection unit **40** may determine a longitudinal distance between the input edge **21** of the multicore fiber **20** and the object **3** to determine whether the object 3 is in near field or in far field. The detection unit **40** may comprise a detection communication utility to transmit data indicative of the longitudinal distance between the input edge **21** of the multicore fiber **20** and the object **3** to the optical assembly controller **50** and/or to operating unit **70.** The optical assembly controller 50 may comprise a controller communication utility to communicate with the detection unit 40 so as to receive data from the detection unit **40** indicative of the longitudinal distance between the input edge **21** of the multicore fiber **20** and the object **3.** The optical assembly controller **50** may be configured to adapt the optical assembly **30** based on said data so as to focus light emitted by the object **3** on the detector array **4.** In other words, the optical assembly controller **50** may be configured to operate the optical assembly **30** so that the light output from the multicore fiber **20** forms an image on the detector array **4** according to a near field or far field configuration of the object **3.** In the embodiment previously mentioned in which the optical assembly **30** is the imaging lens, the optical assembly adapting unit **40** may be configured to displace the imaging lens longitudinally along the optical axis **X** to focus light transferred by the multicore fiber **20** on the detector array **4.** The optical assembly controller **50** may comprise a processor configured to process said data indicative of the longitudinal distance between the input edge **21** of the multicore fiber **20** and the object **3** so as to determine a configuration of the optical assembly **30** for imaging the object **3** according to conjugation relations. In the embodiment in which the optical assembly **30** is the imaging lens, since in respect to its imaging related property, the multicore fiber **20** may actually be regarded as if the input and output edges **21, 22** of the multicore fiber **20** act similarly to principle planes of an lens, the position of the optical assembly **30** may be determined according to the following relation:

$$\frac{1}{U_1 + U_2} + \frac{1}{V} = \frac{1}{F},$$

wherein $U_1$ is the distance between the object **3** and the input edge **21** of the multicore fiber **20**, $U_2$ is the distance between the output edge **22** of the multicore fiber **20,** V is the distance between an optical center of optical assembly **30** and the detection array **4** and F is the focal length of the optical assembly **30.**

The displacing unit **60** (or probe displacing unit) may be configured to shift the input edge **21** of the multicore fiber **20** relatively to the object **3.** In an embodiment, the shift may be performed in a plane substantially perpendicular to the optical axis **X** (so-called "lateral shift") in order to form a set of shifted images of the object **3** on the detector array **4.** Alternatively, or preferably additionally, the shift may be performed along the optical axis (so-called "longitudinal shift"). This may be implemented by the same displacing unit moving the input edge **21** of the multicore fiber **20** along both axis, or additional displacement unit associated with a lens upstream or downstream of the probe.

The probe displacing unit **60** may comprise displacing communication utility configured to receive shifting amplitude instructions and/or shifting direction instructions from the operating unit **70** thereby enabling the operating unit **70** to operate the displacing unit **60.** The operating unit **70** may further comprise a operating communication utility to communicate with the detection unit **40** so as to receive data from the detection unit **40** indicative of the longitudinal distance between the input edge **21** of the multicore fiber **20** and the object **3.** The operating communication utility may further be configured to receive indications from an input utility (not shown) on whether a resolution or a field of view of the imaging is to be improved. The operating unit **70** may further comprise a shift controller configured to set a shifting amplitude to either a first amplitude inferior or equal to the diameter of a core of the multicore fiber **20** or a second amplitude superior or equal to the diameter of the multicore fiber **20.** The setting of the amplitude may for example be based on the distance between the input edge **21** and the object **3** (i.e. a near field mode or far field mode) and on the input from a user received through said input utility.

The processing unit **80** may be connectable to the detector array **4** and configured to acquire and process the set of shifted images formed on the detector array **4** by interlacing said set of shifted images thereby obtaining a combined image of a better resolution or field of view. The operating unit **70** may provide the shifting amplitude set to the processing unit **80.** For near field super resolving used in the present invention, the image processing of interlaced set of shifted images may be performed according to the spatial masking technique disclosed in references [9, 10]. For far field super resolving, the earlier image processing technique developed by the inventor and described in [11] may be used.

Therefore, the present system provides a compact and ergonomic solution simple to manufacture and able to provide images with a high resolution. Further, a user may rely on the operating unit to perform the shifting with a limited amount of manual operations.

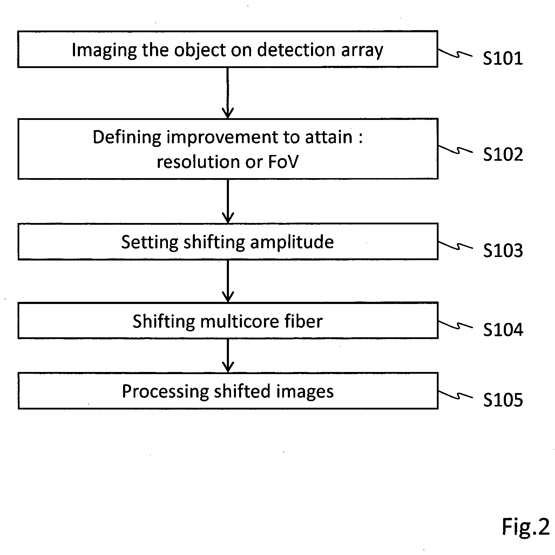

**Fig. 2** illustrates generally steps of a method for imaging an object according to embodiments of the present invention. In a first step **S101,** an image of the object may be formed on a detector array using an optical imaging unit comprising a multicore fiber as an image guide and an optical assembly (for example an imaging lens) to focus light transferred by the multicore fiber on the detector array. The positioning of the optical assembly may be performed based on the position of the object i.e. based on a near field or far field arrangement. The image may further be displayed on a display unit. In a second step **S102,** a user may define an improvement to attain in the image between resolution improvement and field of view improvement, based for example on observation of the displayed image. In a third step **S103,** based on the improvement defined and on whether the object is positioned in the near field or in the far field of the imaging unit, a shifting amplitude may be set between a first amplitude inferior or equal to the diameter of a core of the multicore fiber and a second amplitude superior or equal to the diameter of the multicore fiber.

In the near field mode and to improve resolution, given a predetermined super resolving factor $K$ ($K$ being an integer) to be achieved, the shifting amplitude may be set to the first amplitude. The first amplitude may be determined by the following relation:

$$A_1 = d/K,$$

wherein d is the diameter of the core of the fiber. Further, the number of shifts performed may be equal to the super resolving factor.

In the far field mode and to improve resolution, given the predetermined super resolution factor, the shifting amplitude may be set to the second amplitude. The second amplitude may be determined by the following relation:

$$A_2 = D,$$

wherein D is the diameter of the multicore fiber. Further, the number of shifts may be equal to the super resolving factor.

In order to increase the field of view (and not the resolution) in near field, the shifting may be performed with the second shifting amplitude $A_2$. Particularly, in order to increase the field of view by a factor of K one may perform K shifts with the second shifting amplitude $A_2$. In the far field, in order to increase the field of view by a factor of K one may perform K shifts with the first shifting

amplitude $A_1$.

The position of the object may be detected in order to determine if the object is positioned in the near field or in the far field of the imaging unit. For example, the longitudinal distance between an input edge of the multicore fiber and the object may be detected by a sensor. In an embodiment, the shifting amplitude is set to the second amplitude when (a) the object is in the far field and the resolution is to be improved, and/or (b) the object is in the near field and the field of view to be improved. In an embodiment, the shifting amplitude is set to the first amplitude when (c) the object is in the near field and the resolution is to be improved, and/or (d) the object is in the far field and the field of view is to be improved. Therefore, the same shifting amplitude may be used for different purposes i.e. enhancing resolution or field of view when imaging an object in different modes i.e. in far field and near field mode. In a fourth step **S104,** the multicore fiber input edge may be shifted of said shifting amplitude in order to obtain a set of shifted images. In a fifth step S105, the set of shifted images may be processed to obtain a combined image which attains the improvement defined i.e. a better resolution or field of view than the image obtained in step S101 (also referred to as the original image).

The implementation of the processing of the set of shifted images and the setting of the shifting amplitude based on the relative position of the object with regard to the multicore fiber and on the imaging improvement to attain in the original image may be better understood considering the following:

Any imaging system has limited capability to discriminate between two spatially adjacent features. The physical factors that limit this capability can be divided into two types. The first type is related to the effect of diffraction of light being propagated from the object towards the imaging sensor [8]. The resolution limit due to diffraction as it is obtained in the image plane equals to:

$$\delta_x = 1.22\lambda\, F_\#$$

where $\lambda$ is the optical wavelength and $F_\#$ denotes the F number which is the ratio between the focal length and the diameter of the imaging lens.

The second type is related to the geometry of the detection array [8, 9]. The geometrical limitation can be divided into two kinds of limitations. The first is related to the pitch of the sampling pixels i.e. the distance between two adjacent pixels. This distance determines, according to the Nyquist sampling theorem, the maximal spatial frequency that can be recovered due to spectral aliasing (generated when signals are under sampled in the space domain):

$$\delta_{pitch} = 1/\, 2\upsilon_{max} = 1/BW$$

where $\delta_{pitch}$ is the pitch between adjacent pixels, $\upsilon_{max}$ is the maximal spatial frequency that may be recovered and BW is the bandwidth of the spectrum of the sampled image. The second kind is related to the shape of each pixel and to the fact that each pixel is not a delta function and thus it realizes a non ideal spatial sampling.

In fact, the type of resolution reduction that is being imposed by the multi core probe depends on the distance between the edge of the probe and the object (previously denoted $U_1$).

Diffraction resolution reduction is obtained when the input plane of the probe is relatively away from the object (far field approximation) and then the light distribution on this plane resembles the light distribution over the imaging lens aperture. In that case the diameter of the fiber D sets the maximal spatial frequency transmitted by the fiber and therefore also the spatial resolution obtainable in the image plane:

$$\delta_x = \lambda V/D$$

and the fact that there are multiple cores is equivalent to sampling in the Fourier plane which means replication in the image plane yielding limiting restriction over the obtainable field of view:

$$\Delta_x = \lambda V/d$$

where $\Delta_x$ is the obtainable field of view in the image plane and d is the pitch between two adjacent cores in the multi core probe.

The geometrical limitation is obtained when the distance between the fiber and the object ($U_1$) is relatively small (near field approximation) and then the field of view is limited by the diameter of the fiber D while the pitch between two cores d determine the spatial sampling resolution:

$$\Delta_x = MD$$

and

$$\delta_x = Md$$

where M is the demagnification factor of the proposed imaging system and it equals to:

$$M = V/(U_1 + U_2)$$

The imaging method of the present invention selectively overcomes the geometrical limitation or the diffraction limitation based on detecting whether the object is in near field or in far field and on accordingly setting appropriate shifting amplitude to obtain a set of shifted images thereby enabling to conduct super resolution processing. In super resolution the idea is to encode the spatial information that could not be imaged with the optical system into some other domain. Transmit it through the system and to decode it [8]. The most common domain to do so is the time domain.

Therefore, a way for obtaining resolution improvement in the proposed configuration can be as follows: in the case of far field arrangement when the limiting factor is related to diffraction, the fiber itself can be shifted in time. This time scanning operation will be equivalent to generation of a synthetically increased aperture similar to what happens in synthetic aperture radars (SAR). In this scanning operation the resolution improvement factor is proportional to the ratio between the scanned region and the diameter of the probe D. If instead of super resolution one wishes to increase the imaging field of view, the probe needs to be shifted at amplitude of less than d in order to generate over sampling of spectrum domain by its multiple cores. In this case a set of images are captured while each is obtained after performing a shift of sub core distance. Then, all the images are interlaced together accordingly to generate effective sub core sampling. In the case of near field approximation, temporal scanning once again can improve the resolving capability as described in Refs. [9,10]. In this case the shift is limited by the size of d. Once again a set of images are captured while each is obtained after performing a shift of sub core distance. Then, all the images are interlaced together accordingly to generate effective sub core sampling. In case that instead of resolution improvement one wishes to obtain an increase in the imaging field of view, the probe can again perform scanning but this time at larger amplitude. The field of view enlargement is proportional to the ratio between the shift amplitude and the diameter of the probe D.

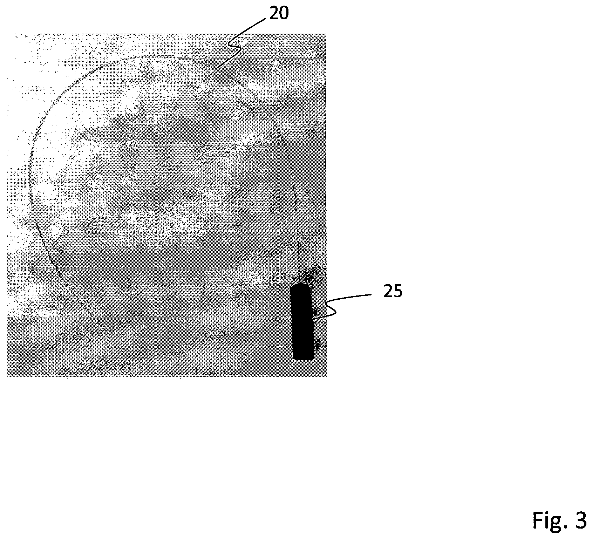

Fig. 3 illustrates a multicore fiber 20 of an imaging system according to an embodiment of the present invention. The multicore fiber 20 may be held at one edge by a holder 25. The multicore fiber 20 may have a large number of optical cores to be sufficient to allow high resolution imaging functionality where the high resolution imaging is performed using the previously described method. The number of fabricated cores in this fiber is about 5,000-10,000. This micro probe is made out of polymers, while the core is made out of PS (Polystyrene) and the cladding is made out of Poly(methyl methacrylate) (PMMA). The refractive indexes of the PS and the PMMA at a wavelength of 632nm are 1.59 and 1.49, respectively.

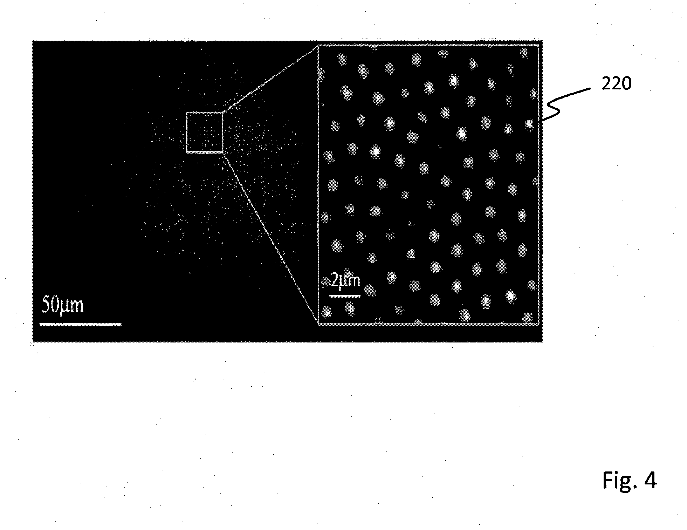

Fig. 4 presents the multi cores 220 visible from the output edge of the probe transferring light emitted by an object. The probe presented in Fig. 4 has about 5,000 cores but the actual spatial resolution even with this number of cores might be larger due to super resolution processing that is to be elaborated according to the method previously described. In the figure one may see that thousands of individual light channels transmitting spatial information at wavelength of 630nm. Each channel may basically be a different pixel in the constructed image. As previously mentioned, the fact that each pixel may be transmitted individually and the endoscope may basically be a multi core fiber rather than a multi mode fiber allows generating an image that is insensitive to bending of the fiber. This feature enables the multicore fiber to be bent and to perform for example an endoscopy procedure.

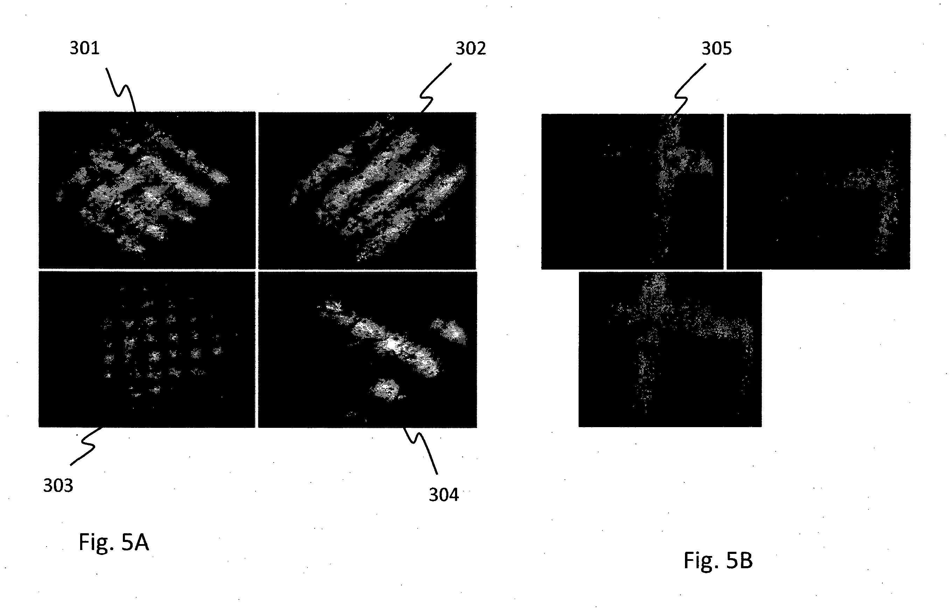

**Fig. 5A** and **Fig. 5B** present experimental results of images acquired by an imaging system according to an embodiment of the invention. Fig. 5A presents high resolution target imaging with the micro probe of Fig. 3. The presented experimental results include images that were transmitted backwards by the multicore fiber and imaged on a detector array (CCD camera). The imaged object was a resolution target while the presented images include in the upper line from left to right: an object **301** with rotated black vertical lines and an object **302** with rotated black vertical lines rotated at 90 degrees with respect to object **301.** In the second row form left to right we have an object **303** with small black rectangles and then an object **304** with large black lines and black rectangle appearing in the left side of the backwards transmitted image. Fig. 5B shows three shifted images of a square like shape resolution target **305** imaged at different locations i.e. by shifting the input edge of the multicore fiber with regard to the square like shape resolution target **305** using an imaging system according to an embodiment of the present invention including the multicore fiber shown on Fig. 3. This figure demonstrates that although the multicore fiber (micro probe) is very thin in its diameter, a large field of view may be obtained by proper cascading the acquired shifted images.

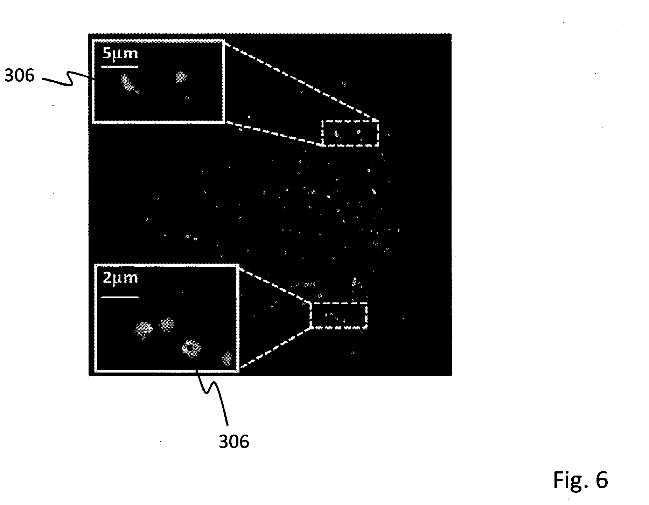

**Fig. 6** presents experimental results of images of Fe beads **306** having diameter of around $1\mu m$ imaged through an agar solution. In order to further demonstrate the high spatial discrimination the experiment is repeated. The imaging of the Fe micro beads **306** is performed through agar solution in order to show that imaging through biological like medium is possible and to demonstrate the high spatial resolution of the fabricated prototype allowing spatial separation between sub micron features. The experimental results presented in Fig. 6 were performed by using an imaging system according to an embodiment of the invention including the multicore fiber shown on Fig. 3.

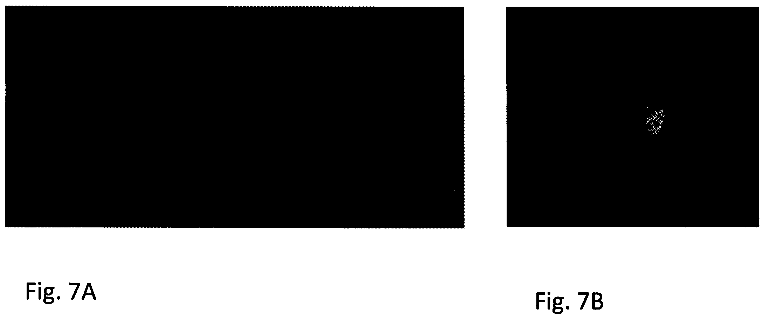

**Fig. 7A** and **Fig. 7B** present imaging of rat heart muscle growth on a slide. Fig. 7Ashows a top view microscope image of the rat heart muscle, while Fig. 7B shows these cells (shown in the inset image of Fig. 7A) imaged with tan imaging system according to an embodiment of the present invention including the multicore fiber shown on Fig. 3. Cell culture and sample preparation for measurements was as follows: Rat cardiac myocytes were isolated. Briefly, hearts from newborn rats were rinsed in phosphate buffered saline (PBS), cut into small pieces and incubated with a solution of proteolytic enzymes-RDB (Biological Institute, Ness-Ziona, Israel). Separated cells were suspended in Dulbecco's Modified Eagle's Medium (DMEM) containing 10% inactivated horse serum (Biological Industries, Kibbutz Beit Haemek, Israel) and 2% chick embryo extract, and centrifuged at $300 \times g$ for 5 min. Precipitant (cell pellet) was resuspended in growth medium and placed in culture dishes on collagen/gelatin coated cover-glasses. On day 4, cells were treated with 0.5-5 $\mu M$ of DOX for 18 hours and then with drug-free growth medium for an additional 24 hours. Cell samples were grown on microscope cover slips and imaged by an imaging system according to an embodiment of the present invention including the multicore fiber shown on Fig. 3.

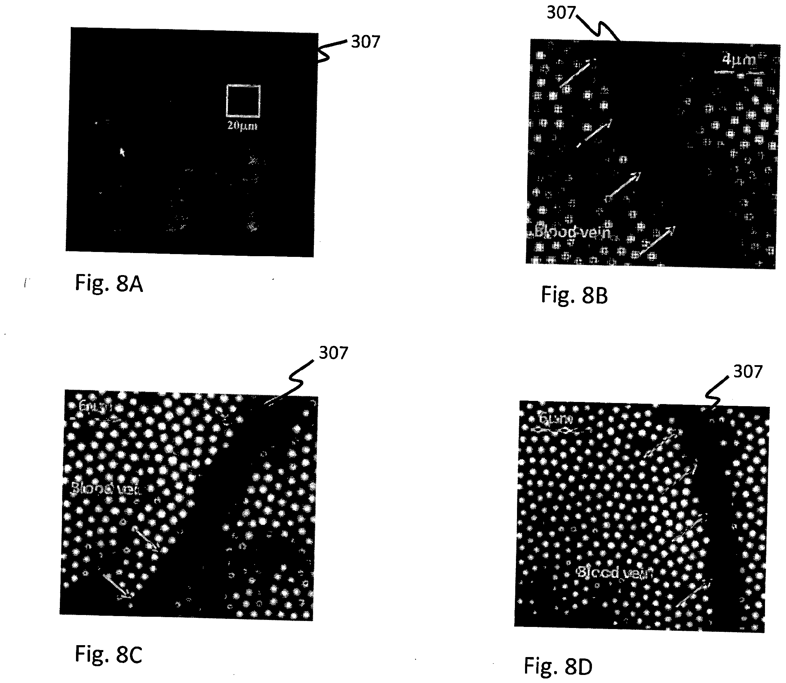

**Fig. 8A-8D** present imaging of blood veins inside a chicken wing. Fig. 8Ashows a top view microscope image of veins **307** of the chicken wing area, while Figs. 8B-8D show the veins **307** (indicated by solid arrows) imaged with an imaging system according to an embodiment of the invention including the multicore fiber shown on Fig. 3.

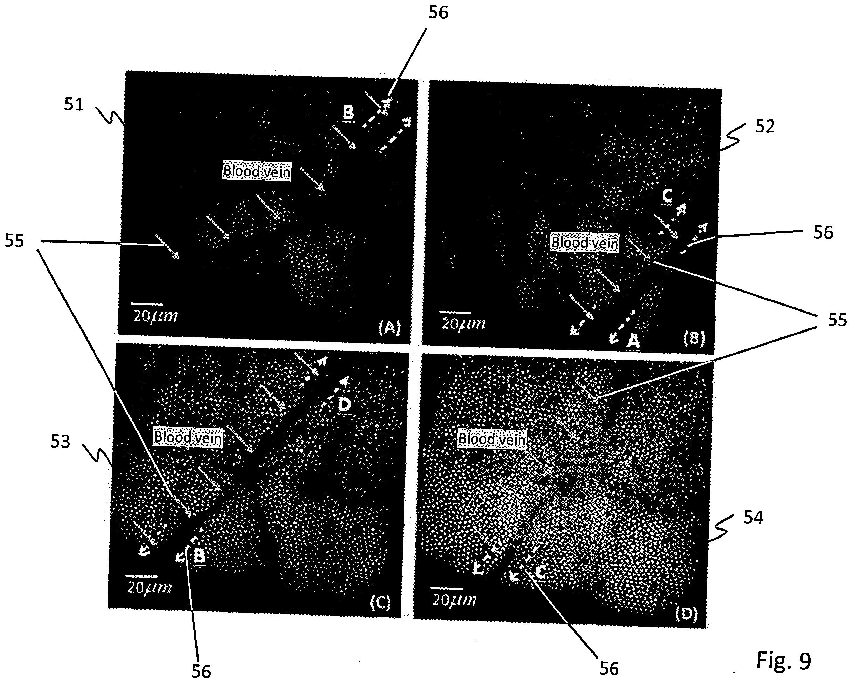

**Fig. 9** illustrates experimental results of imaging along a chicken wing with an imaging system according to an embodiment of the present invention including the multicore fiber shown on Fig. 3. Images **51-54** show shifted images of a blood vein (shown with solid arrows) in the chicken wing. Images **51-54** have been taken by shifting the input edge of the multicore fiber. Although, the multicore fiber (micro probe) diameter is equal to $200\mu m$, a construction of a large image can be obtained with real time image processing. This can be done by calculating the shift (or relative movement) of the probe displacing unit (in the present example, a multi axis platform where the micro probe is located) and converting it to the image movement. Each one of the images **51-54** shows different location of the micro probe along the imaged blood vein. In fact, from the visualization point of view, the images are individually shown instead of being cascaded into a single image. The solid arrows **55** indicate the blood vein, while the dashed arrows as well as the labeling letter indicate the cascading point between the images presented in Fig. 9 The four images **51-54** correspond to the references A̲, B̲, C̲ and D̲ shown on **Fig. 9** and in each of images **51-54,** the dashed lines **56** indicate in which position in respect to a given image the other images appear. So each of the four images is sectioned in a larger field of view. By proper cascading the images a full length of the examined area of interest (e.g. blood vein) can be obtained. Such cascading may also be referred to as a mosaicing or an image processing to improve field of view.

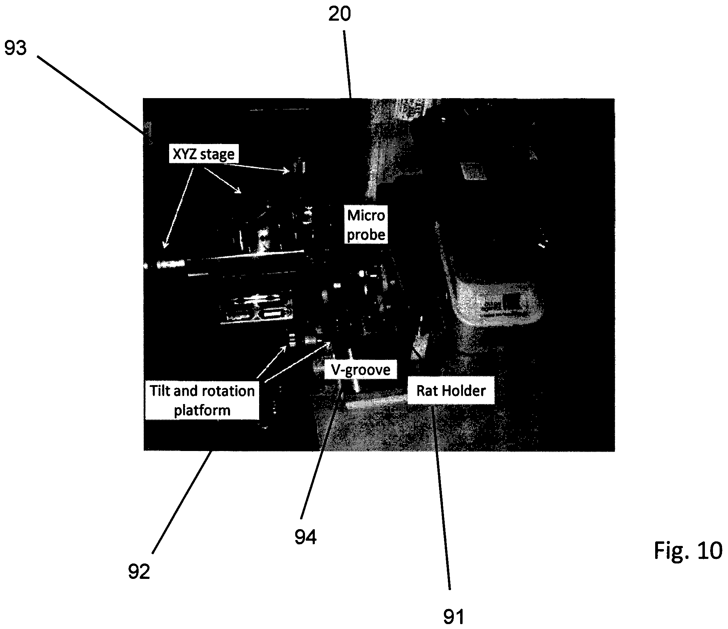

**Fig. 10** shows the experimental setup used for imaging from inside a brain of a rat. The setup includes a first module and a second module. The first module comprises a special rat holder 91 that is used to hold the rat during the surgery and the experiments processes and the second module is related to a probe displacing unit (platform) comprising a tilt and rotation platform 92, an XYZ stage 93 and a V groove 94 that enables accurate navigation of the micro probe 20 inside the examined area. Navigating the multicore fiber (micro probe) inside the brain tissue may be done by using the probe displacing unit which provides five axis positioning stage through the XYZ stage 93 and the tilt and rotation platform 92. On top of the multi axis stage the V-groove is configured as a bare fiber holder which allows locating the micro probe at an examined area.

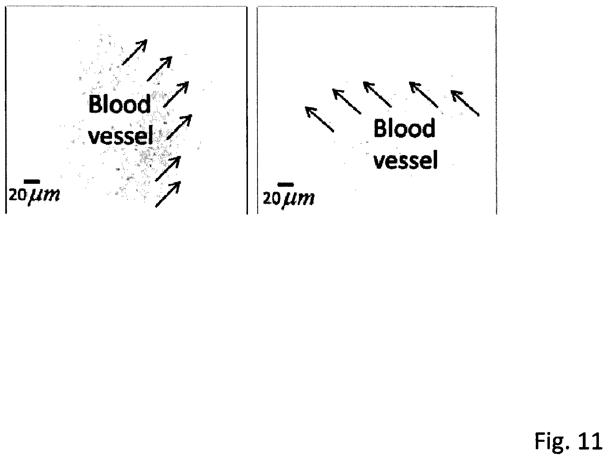

**Fig. 11** is a picture with inversed colors showing blood veins (indicated by solid arrows) of rat brain imaged by an imaging system according to the present invention including the multicore fiber shown on Fig. 3 and using the experimental setup described on Fig. 10. Imaging is performed by penetrating the micro probe inside the brain tissue. In brief the surgical procedure was as follows: the rat has been placed in a cage where isofleurane is injected through a vaporizior. Right after, the rat is anaesthetised using injection of urethane and positioned at a special rat head holder. Then an aproximettly 7 mm diameter hole is drilled above the barrel cortex of the rat, which is identified by the anatomical coordinates. The dura is gentelly removed to perform the experiments procedure.Note that the proposed probe that is being realized is very thin in its diameter in comparison to commonly used endoscope, while it does not provide only spatial resolution that is as good but also enable both penetration inside the examined area and minimal invasive damage as well.

The above examples and description have of course been provided only for the purpose of illustration, and are not intended to limit the invention in any way. As will be appreciated by the skilled person, the invention can be carried out in a great variety of ways, employing more than one technique from those described above, all without exceeding the scope of the invention, as defined in the appended claims.

**Claims**

1.    A system (1) for imaging an object (3) comprising:

- an optical imaging unit (2) defining an optical axis and comprising a multicore fiber (20) configured to collect light from the object (3) at an input edge (21) of the multicore fiber and transfer the collected light to an output edge (22) of the multicore fiber, wherein the multicore fiber has a diameter D, and a plurality of cores having a pitch *d;*
- a detector array (4) configured to have formed thereon, by the optical imaging unit, an image of the object;
- a detection unit (40) configured to detect a longitudinal distance between the object and the input edge of the multicore fiber, and to determine thereby if the object is in a near field or a far field of the optical imaging unit;
- a displacing unit (60) configured to shift the input edge of the multicore fiber relative to the object in a plane substantially perpendicular to the optical axis and along the optical axis, forming thereby a set of shifted images of the object on the detector array;
- an operating unit (70) configured to: receive data from the detection unit indicative of the longitudinal distance between the object and the input edge of the multicore fiber; and operate the displacing unit by setting a shifting amplitude to either a first shifting amplitude inferior or equal to the diameter of a core of the multicore fiber or a second shifting amplitude superior or equal to the diameter of the multicore fiber;
- a processing unit (80) connectable to the detection array and configured to receive and process data indicative of the set of shifted images, and to interlace one or more of said shifted images to obtain a combined image,

wherein:
the operating unit comprises a shift controller configured to set the shifting amplitude based on the longitudinal distance between the input edge and the object to either: the first shifting amplitude inferior or equal to the diameter of a core of the multicore fiber; or the second shifting amplitude superior or equal to the diameter *D* of the multi core fiber.

2.    The system according to claim 1, wherein the optical imaging unit comprises an optical assembly (50) configured to collect light from the output edge of the multicore fiber and form an image of the object on the detection array.

3.    The system according to claim 1 or 2, wherein the optical imaging unit comprises a lens unit arranged upstream of the input edge of the multicore fiber with respect to a direction of light propagation through the system, said lens unit being displaceable along the optical axis with respect to the object.

4.    The system according to any one of the preceding claims, wherein the operating unit comprises an input utility configured to receive input from a user defining whether the field of view or resolution of the imaging is to be improved.

5.    The system according to claim 4, wherein:

the shifting amplitude is set to the second amplitude when:

(a) the object is in the far field and the resolution is to be improved; and/or
(b) the object is in the near field and the field of view is to be improved; and

the shifting amplitude is set to the first amplitude when:

(c) the object is in the near field and the resolution is to be improved; and/or
(d) the object is in the far field and the field of view is to be improved

6. The system according to any one of the preceding claims, wherein the multicore fiber is either a fiber bundle or a photonic crystal.

7. The system according to any one of the preceding claims, wherein the multicore fiber has a polygonal cross section defining two opposite substantially parallel facets.

8. The system according to claim 7, further comprising electrodes located on said opposite facets of the multicore fiber to carry out at least one of electrical stimulation and sensing temperature using the Peltier effect.

9. The system according to claim 1, further comprising:

- a coherent light source configured to illuminate the object and provide a reference wave front; and
- an holographic or interferometric setup configured to provide interference between the reference wave front and a reflected wave front reflected by the object and transferred by the multicore fiber, thereby providing phase information on light reflected by the object.

10. A method for imaging an object comprising:

- transferring light coming from the object through a multicore fiber having an input edge and an output edge;
- imaging the object on a detection array by collecting, by an imaging optical unit, light from the output edge of the multicore fiber;
- detecting, by a detection unit, a longitudinal distance between the object and the input edge of the multicore fiber, and determining thereby if the object is in a near field or a far field of the optical imaging unit;
- setting, by a shift controller of an operating unit, a shifting amplitude for the multicore fiber based on the longitudinal distance between the input edge and the object, such that the shifting amplitude is either a first shifting amplitude inferior or equal to the diameter of a core of the multicore fiber or a second shifting amplitude superior or equal to the diameter of the multicore fiber, to enable improvement of either resolution or the field of view of imaging;
- shifting, by a displacement unit, the input edge of the multicore fiber along, and in a plane substantially perpendicular to, an axis of light propagation from the object to the detection array, using said shifting amplitude, thereby obtaining a set of shifted images of the object; and
- processing, by a processing unit, said set of shifted images in order to obtain a combined image of the object by interlacing said shifted images for improving the resolution or field of view.

11. The method according to claim 10, comprising moving a lens unit, in front of the input edge of the multicore fiber, along said axis of light propagation, with respect to the object.

**Patentansprüche**

1. System (1) zum Abbilden eines Objekts (3), umfassend:

- eine optische Bildgebungseinheit (2), die eine optische Achse definiert und eine Multicore-Faser (20) umfasst, die dazu konfiguriert ist, Licht von einem Objekt (3) an einer Eingangskante (21) der Multicore-Faser zu sammeln und das gesammelte Licht auf eine Ausgangskante (22) der Multicore-Faser zu übertragen, wobei die Multicore-Faser einen Durchmesser D und eine Vielzahl von Adern eine Steigung $d$ hat;
- eine Detektoranordnung (4), die dazu konfiguriert ist, mittels der optischen Bildgebungseinheit ein Bild des Objekts hierauf ausgebildet zu haben;
- eine Erfassungseinheit (40), die dazu konfiguriert ist, eine Längsentfernung zwischen dem Objekt und der Eingangskante der Multicore-Faser zu erfassen und hierdurch zu bestimmen, ob das Objekt in einem Nahfeld oder Fernfeld der optischen Bildgebungseinheit ist;
- eine Verschiebungseinheit (60), die dazu konfiguriert ist, die Eingangskante der Multicore-Faser in Bezug auf das Objekt in einer Ebene, die im Wesentlichen senkrecht zu der optischen Achse und entlang der optischen Achse ist, zu verlagern, wodurch ein Satz verlagerter Bilder des Objekts auf der Detektoranordnung ausgebildet wird;
- eine Betriebseinheit (70), die hierzu konfiguriert ist: zum Empfangen von Daten von der Erfassungseinheit, die einen Hinweis auf die Längsentfernung zwischen dem Objekt und der Eingangskante der Multicore-Faser geben; und zum Betreiben der Verschiebungseinheit durch Einstellen einer Verlagerungsamplitude entweder auf eine erste Verlagerungsamplitude, die geringer oder gleich dem Durchmesser einer Ader der Multicore-Faser ist, oder eine zweite Verlagerungsamplitude, die höher oder gleich dem Durchmesser der Multicore-Faser ist;
- eine Verarbeitungseinheit (80), die mit der Erfassungsanordnung verbindbar ist und dazu konfiguriert ist, Prozessdaten, die einen Hinweis auf den Satz verlagerter Bilder geben, zu empfangen und eines oder mehr der verlagerten Bilder zu verschachteln, um ein Kombinationsbild zu erhalten, wobei die Betriebseinheit einen Verlagerungscontroller umfasst, der dazu konfiguriert ist, die Verlagerungsamplitude basierend auf der Längsentfernung zwischen der Eingangskante und dem Objekt einzustellen, um entweder die erste Verlagerungsamplitude, die geringer oder gleich dem Durchmesser einer Multicore-Faser oder die zweite Verlagerungsamplitude, die höher oder gleich dem Durchmesser D der Multicore-Faser ist, einzustellen.

2. System nach Anspruch 1, wobei die optische Bildgebungseinheit eine optische Baugruppe (50) umfasst, die dazu konfiguriert ist, Licht von der Ausgangskante der Multicore-Faser zu sammeln und ein Bild auf der Erfassungsanordnung auszubilden.

3. System nach Anspruch 1 oder 2, wobei die optische Bildgebungseinheit eine Linseneinheit umfasst, die der Eingangskante der Multicore-Faser in Bezug auf eine Richtung der Lichtausbreitung durch das System vorgeschaltet ist, und wobei diese Linseneinheit in Bezug auf das Objekt entlang der optischen Achse verschiebbar ist.

4. System nach einem der vorhergehenden Ansprüche, wobei die Betriebseinheit ein Eingangsdienstprogramm umfasst, das dazu konfiguriert ist, einen Eingang von einem Nutzer zu empfangen, wodurch definiert ist, ob das Sichtfeld oder die Auflösung der Bildgebung zu verbessern ist.

5. System nach Anspruch 4, wobei die Verlagerungsamplitude auf die zweite Amplitude eingestellt ist, wenn:

   (a) das Objekt in dem Fernfeld ist und die Auflösung zu verbessern ist; und/oder
   (b) das Objekt in dem Nahfeld ist und das Sichtfeld zu verbessern ist; und die Verlagerungsamplitude ist auf die erste Amplitude eingestellt, wenn:
   (c) das Objekt in dem Nahfeld ist und die Auflösung zu verbessern ist; und/oder
   (d) das Objekt in dem Fernfeld ist und das Sichtfeld zu verbessern ist.

6. System nach einem der vorhergehenden Ansprüche, wobei die Multicore-Faser entweder ein Lichtfaserbündel oder ein photonisches Kristall ist.

7. System nach einem der vorhergehenden Ansprüche, wobei die Multicore-Faser einen polygonalen Querschnitt hat, der zwei einander gegenüberliegende im Wesentlichen parallele Facetten definiert.

8. System nach Anspruch 7, ferner umfassend Elektroden, die an den einander gegenüberliegenden Facetten der Multicore-Faser lokalisiert sind, um durch Verwendung des Peltier-Effekts mindestens eines von elektrischer Stimulierung und Temperaturfühlen durchzuführen.

9. System nach Anspruch 7, ferner umfassend:

   - eine kohärente Lichtquelle, die dazu konfiguriert ist, das Objekt zu beleuchten und eine Referenzwellenfront bereitzustellen; und
   - ein holografisches oder interferometrisches Setup, das dazu konfiguriert ist, eine Interferenz zwischen der Referenzwellenfront und einer reflektierten Wellenfront, die von dem Objekt reflektiert und von der Multicore-Faser übertragen wird, bereitzustellen, wodurch Phaseninformationen zum Licht, das von dem Objekt reflektiert wird, bereitgestellt wird.

10. Verfahren zum Abbilden eines Objekts, umfassend:

   - Übertragen von Licht, das von dem Objekt kommt, mittels einer Multicore-Faser, die eine Eingangs- und eine Ausgangskante hat;
   - Bildgebung des Objekts auf einer Erfassungsanordnung, indem mittels einer optischen Bildgebungseinheit Licht von der Ausgangskante der Multicore-Faser gesammelt wird;
   - Erfassen einer Längsentfernung zwischen dem Objekt und der Eingangskante der Multicore-Faser mittels einer Erfassungseinheit und hierdurch Bestimmen ob das Objekts in einem Nahfeld oder einem Fernfeld der optischen Bildgebungseinheit ist;
   - Einstellen einer Verlagerungsamplitude für die Multicore-Faser durch einem Verlagerungscontroller einer Betriebseinheit basierend auf der Längsentfernung zwischen der Eingangskante und dem Objekt, derart, dass die Verlagerungsamplitude entweder eine erste Verlagerungsamplitude ist, die geringer oder gleich dem Durchmesser einer Ader der Multicore-Faser ist, oder eine zweite Verlagerungsamplitude, die höher oder gleich dem Durchmesser der Multicore-Faser ist, um eine Verbesserung entweder der Auflösung oder des Sichtfelds der Bildgebung zu ermöglichen;

- Verlagern der Eingangskante der Multicore-Faser durch eine Verlagerungseinheit entlang und in einer Ebene, die im Wesentlichen senkrecht zu einer Achse der Lichtausbreitung von dem Objekt zu der Erfassungsanordnung ist, wobei die Verlagerungsamplitude dazu verwendet wird, um hierdurch einen Satz verlagerter Bilder des Objekts zu erhalten, und
- Verarbeiten des Satzes verlagerter Bilder mittels einer Verarbeitungseinheit, um durch Verschachteln der verlagerten Bilder ein Kombinationsbild des Objekts zu erhalten, damit die Auflösung des Sichtfelds verbessert wird.

11. Verfahren nach Anspruch 10, umfassend Bewegen einer Linseneinheit vorn vor die Eingangskante der Multicore-Faser in Bezug auf das Objekt entlang der Achse der Lichtausbreitung.

**Revendications**

1. Système (1) d'imagerie d'un objet (3) comprenant :

   o une unité d'imagerie optique (2) définissant un axe optique et comprenant une fibre multicœur (20) configurée pour collecter la lumière de l'objet (3) au niveau d'un bord d'entrée (21) de la fibre multicœur et transférer la lumière collectée vers un bord de sortie (22) de la fibre multicœur, la fibre multicœur ayant un diamètre D, et une pluralité de cœurs ayant un pas *d* ;
   o un réseau de détecteurs (4) configuré pour que l'unité d'imagerie optique forme une image de l'objet sur celui-ci ;
   o une unité de détection (40) configurée pour détecter une distance longitudinale entre l'objet et le bord d'entrée de la fibre multicœur, et pour déterminer ainsi si l'objet est dans un champ proche ou un champ éloigné de l'unité d'imagerie optique ;
   o une unité de déplacement (60) configurée pour décaler le bord d'entrée de la fibre multicœur par rapport à l'objet dans un plan sensiblement perpendiculaire à l'axe optique et le long de l'axe optique, formant ainsi un ensemble d'images décalées de l'objet sur le réseau de détecteurs ;
   o une unité d'exploitation (70) configurée pour : recevoir des données de l'unité de détection indicatives de la distance longitudinale entre l'objet et le bord d'entrée de la fibre multicœur ; et faire fonctionner l'unité de déplacement en fixant une amplitude de décalage soit à une première amplitude de décalage inférieure ou égale au diamètre d'un cœur de la fibre multicœur soit à une seconde amplitude de décalage supérieure ou égale au diamètre de la fibre multicœur ;
   o une unité de traitement (80) pouvant être connectée au réseau de détection et configurée pour recevoir et traiter des données indicatives de l'ensemble d'images décalées, et pour interfacer une ou plusieurs desdites images décalées afin d'obtenir une image combinée,

   dans lequel :
   l'unité d'exploitation comprend un contrôleur de décalage configuré pour régler l'amplitude de décalage sur la base de la distance longitudinale entre le bord d'entrée et l'objet afin que : la première amplitude de décalage soit inférieure ou égale au diamètre d'un noyau de la fibre multicœur ; ou que la deuxième amplitude de décalage soit supérieure ou égale au diamètre D de la fibre multicœur.

2. Système selon la revendication 1, dans lequel l'unité d'imagerie optique comprend un ensemble optique (50) configuré pour collecter la lumière du bord de sortie de la fibre multicœur et former une image de l'objet sur le réseau de détection.

3. Système selon la revendication 1 ou 2, dans lequel l'unité d'imagerie optique comprend une unité de lentille disposée en amont du bord d'entrée de la fibre multicœur par rapport à une direction de propagation de la lumière à travers le système, ladite unité de lentille étant déplaçable le long de l'axe optique par rapport à l'objet.

4. Système selon l'une quelconque des revendications précédentes, dans lequel l'unité d'exploitation comprend un utilitaire d'entrée configuré pour recevoir une entrée d'un utilisateur définissant si le champ de vision ou la résolution de l'imagerie doit être amélioré.

5. Système selon la revendication 4, dans lequel :

   l'amplitude de décalage est fixée à la deuxième amplitude lorsque :

      (a) l'objet est dans le champ lointain et la résolution doit être améliorée ; et/ou
      (b) l'objet est dans le champ proche et le champ de vision doit être amélioré ; et

   l'amplitude de décalage est fixée à la première amplitude lorsque :
   (c) l'objet est dans le champ proche et la résolution doit être améliorée ; et/ou
   (d) l'objet est dans le champ lointain et le champ de vision doit être amélioré.

6. Système selon l'une quelconque des revendications précédentes, dans lequel la fibre multicœur est soit un faisceau de fibres soit un cristal photonique.

**7.** Système selon l'une quelconque des revendications précédentes, dans lequel la fibre multicœur présente une section polygonale définissant deux facettes opposées sensiblement parallèles.

**8.** Système selon la revendication 7, comprenant en outre des électrodes situées sur lesdites facettes opposées de la fibre multicœur afin d'effectuer au moins une stimulation électrique et une détection de température en utilisant l'effet Peltier.

**9.** Système selon la revendication 1, comprenant en outre :

o une source de lumière cohérente configurée pour éclairer l'objet et fournir un front d'onde de référence ; et

o une configuration holographique ou interférométrique configurée pour fournir une interférence entre le front d'onde de référence et un front d'onde réfléchi par l'objet et transféré par la fibre multicœur, fournissant ainsi des informations de phase sur la lumière réfléchie par l'objet.

**10.** Procédé d'imagerie d'un objet consistant à :

o transférer la lumière provenant de l'objet à travers une fibre multicœur ayant un bord d'entrée et un bord de sortie ;

o imager l'objet sur un réseau de détection en collectant, par une unité optique d'imagerie, la lumière provenant du bord de sortie de la fibre multicœur ;

o détecter, par une unité de détection, une distance longitudinale entre l'objet et le bord d'entrée de la fibre multicœur, et déterminer ainsi si l'objet est dans un champ proche ou un champ éloigné de l'unité d'imagerie optique ;

o régler, par un contrôleur de décalage d'une unité d'exploitation, une amplitude de décalage pour la fibre multicœur en fonction de la distance longitudinale entre le bord d'entrée et l'objet, de sorte que l'amplitude de décalage soit une première amplitude de décalage inférieure soit égale au diamètre d'un cœur de la fibre multicœur, ou une seconde amplitude de décalage supérieure ou égale au diamètre de la fibre multicœur, afin de permettre l'amélioration soit de la résolution, soit du champ de vision de l'imagerie ;

o décaler, par une unité de déplacement, le bord d'entrée de la fibre multicœur le long et dans un plan sensiblement perpendiculaire à un axe de propagation de la lumière de l'objet au réseau de détection, en utilisant ladite amplitude de décalage, obtenant ainsi un ensemble d'images décalées de l'objet ; et

o traiter, par une unité de traitement, ledit ensemble d'images décalées afin d'obtenir une image combinée de l'objet en interfaçant lesdites images décalées afin d'améliorer la résolution ou le champ de vision.

**11.** Procédé selon la revendication 10, comprenant le déplacement d'une unité de lentille, devant le bord d'entrée de la fibre multicœur, le long dudit axe de propagation de la lumière, par rapport à l'objet.

Fig.1

EP 2 661 211 B1

```
┌─────────────────────────────────────────────┐
│      Imaging the object on detection array    │ ~~ S101
└─────────────────────────────────────────────┘
                      │
                      ▼
┌─────────────────────────────────────────────┐
│         Defining improvement to attain :      │ ~~ S102
│               resolution or FoV               │
└─────────────────────────────────────────────┘
                      │
                      ▼
┌─────────────────────────────────────────────┐
│            Setting shifting amplitude         │ ~~ S103
└─────────────────────────────────────────────┘
                      │
                      ▼
┌─────────────────────────────────────────────┐
│             Shifting multicore fiber          │ ~~ S104
└─────────────────────────────────────────────┘
                      │
                      ▼
┌─────────────────────────────────────────────┐
│             Processing shifted images         │ ~~ S105
└─────────────────────────────────────────────┘
```

Fig.2

Fig. 3

Fig. 4

Fig. 5A

Fig. 5B

Fig. 6

Fig. 7B

Fig. 7A

Fig. 8A

Fig. 8B

Fig. 8C

Fig. 8D

Fig. 9

XYZ stage

Micro probe

Tilt and rotation platform

V-groove

Rat Holder

20

93

92

94

91

Fig. 10

EP 2 661 211 B1

Fig. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100046897 A1
- US 2010274090
- US 2010282954
- US 2008081950 A1

**Non-patent literature cited in the description**

- **G. UNFRIED1 ; F. WIESER ; A. ALBRECHT ; A. KAIDER ; F. NAGELE.** Flexible versus rigid endoscopes for outpatient hysteroscopy: a prospective randomized clinical trial. *Human Reproduction,* 2001, vol. 16, 168-171
- **R. P. J. BARRETTO ; B. MESSERSCHMIDT ; M. J. SCHNITZER.** In vivo fluorescence imaging with high-resolution microlenses. *Nature methods,* 2009, vol. 6, 511-514
- **B. A. FLUSBERG ; E. D. COCKER ; W. PIYAWATTANAMETHA ; J. C. JUNG ; E. L. M. CHEUNG ; M. J. SCHNITZER.** Fiber-optic fluorescence imaging. *Nature methods,* 2005, vol. 2, 941-950
- **M. E. LLEWELLYN ; R. P. J. BARRETTO ; S. L. DELP ; M. J. SCHNITZER.** Minimally invasive high-speed imaging of sarcomere contractile dynamics in mice and humans. *Nature,* 2008, vol. 454, 784-788
- **K. DEISSEROTH ; G. FENG ; A. K. MAJEWSKA ; G. MIESENBOCK ; A. TING ; M. J. SCHNITZER.** Next-Generation Optical Technologies for Illuminating Genetically Targeted Brain Circuits. *The Journal of Neuroscience,* 2006, vol. 26, 10380-10386
- **B. A. FLUSBERG ; J. C. JUNG ; E. D. COCKER ; E. P. ANDERSON ; M. J. SCHNITZER.** In vivo brain imaging using a portable 3.9 gram two-photon fluorescence microendoscope. *Opt. Lett.,* 2005, vol. 30, 2272-2274
- **W. PIYAWATTANAMETHA ; E. D. COCKER ; L. D. BURNS ; R. P. J. BARRETTO ; J. C. JUNG ; H. RA ; O. SOLGAARD ; M. J. SCHNITZER.** In vivo brain imaging using a portable 2.9g two-photon microscope based on a microelectromechanical systems scanning mirror. *Opt. Lett.,* 2009, vol. 34, 2309-2311
- **Z. ZALEVSKY ; D. MENDLOVIC.** Optical Super Resolution. Springer, 2004
- **A. BORKOWSKI ; Z. ZALEVSKY ; B. JAVIDI.** Geometrical Super Resolved Imaging Using Non periodic Spatial Masking. *JOSA A,* 2009, vol. 26, 589-601
- Evaluation of the microscanning process. **J. FORTIN ; P. CHEVRETTE ; R. PLANTE.** SPIE. 1994, vol. 2269, 271-279
- **V. MICO ; Z. ZALEVSKY ; J. GARCIA.** Common-path Phase-shifting Digital Holographic Microscopy: a way to Quantitative Phase Imaging and Superresolution. *Opt. Commun.,* 2008, vol. 281, 4273-4281